# EUROPÄISCHE PATENTSCHRIFT

(11) **EP 2 337 496 B1**
(45) Veröffentlichungstag und Bekanntmachung des Hinweises auf die Patenterteilung: **27.08.2014**
(21) Anmeldenummer: 09775739.7
(22) Anmeldetag: 07.07.2009
(51) Int. Cl.: A61B 5/1459

(54) **VORRICHTUNG ZUR MESSUNG DER DURCHBLUTUNG IN EINEM KÖRPERGEWEBE**
DEVICE FOR MEASURING THE BLOOD FLOW OF A BODY TISSUE
DISPOSITIF SERVANT À MESURER L'IRRIGATION SANGUINE D'UN TISSU CORPOREL

(30) Priorität: 06.08.2008 CH 12292008
(43) Veröffentlichungstag der Anmeldung: 29.06.2011
(73) Patentinhaber: Carag AG, 6340 Baar (CH)
(72) Erfinder: LIMACHER, Kuno, CH-6312 Steinhausen (CH); BERNHARD, Jérôme, CH-8002 Zürich (CH); HUMMEN, Jörg, CH-8802 Kilchberg (CH); MANNHART, Jevgenij, CH-6330 Cham (CH); STEINER, Claudio, CH-6430 Schwyz (CH)
(74) Vertreter: Clerc, Natalia
(86) Internationale Anmeldenummer: PCT/CH2009/000241
(87) Internationale Veröffentlichungsnummer: WO 2010/015094

(56) Entgegenhaltungen:
- EP-A1- 1 149 604
- CH-B1- 697 482
- US-A- 5 643 226
- US-A1- 2004 111 016
- US-A1- 2006 253 007
- US-A1- 2008 004 511
- US-B1- 6 697 667

## Beschreibung

### Technisches Gebiet der Erfindung

Die vorliegende Erfindung betrifft einen Katheter und eine Vorrichtung zur Messung der Durchblutung in einem Körpergewebe, insbesondere einem zerebralen Gewebe.

### Stand der Technik

Aus dem Stand der Technik sind diverse Einrichtungen zur Messung der Durchblutung, bzw. des Blutdurchflusses durch ein Körpergewebe bekannt. Für die Messung der Durchblutung im zerebralen Gewebe des Gehirns kommen Kathetermessvorrichtungen zum Einsatz, die an ihrer Katheterspitze Messsensoren tragen. Solche Kathetersonden werden durch eine in der Schädeldecke vorbereitete Öffnung in das zerebrale Gewebe eingeführt, um dort eine Durchflussmessung durchzuführen. Hierfür sind mehrere Messverfahren bekannt, beispielhaft werden hier die Thermodiffusion, Ultraschallverfahren und die Nahinfrarot-Spektroskopie in Verbindung mit einem Indikator genannt.

Aus der EP 1 464 276 A1 ist beispielsweise eine Messvorrichtung zur Bestimmung des Blutflusses bekannt, bei der auf der Oberfläche des Kopfes zwei Oktoden in einem Abstand zueinander befestigt werden. Eine der Oktoden ist mit einer Strahlungsquelle verbunden, die Strahlung mit nahinfraroter Wellenlänge aussendet. An der zweiten Oktode tritt ein an dem zerebralen Gewebe reflektierter Teil der Strahlung ein, so dass die Intensität durch eine Auswerteeinheit ermittelt werden kann. Als Indikator wird Indocyanin-Grün verwendet und es wird ein Lichtstrahl einer Wellenlänge zwischen 780 und 910 nm verwendet. Bei dieser Art der Durchführung einer Nahinfrarot-Spektroskopiemessung muss mit einer Vielzahl von äusseren Einflüssen gerechnet werden, welche die Messung des Blutdurchflusses nachteilig beeinflussen. Der Lichtstrahl kann nicht unmittelbar zu dem zu messenden Gewebe geführt werden, sondern muss zunächst durch die Haut, die Schädelkalotte, die Dura mater, usw. hindurch zu dem Untersuchungsgewebe gelangen. Dabei wird das Messsignal z. B. durch Absorption und Streuung abgeschwächt und verfälscht. Es können daher nur Gewebebereiche nahe der Oberfläche des Kopfes vermessen werden. Bereiche im Inneren des Gehirns, wie zum Beispiel nahe des Ventrikelbodens, können mit diesem Verfahren nur unzureichend genau erfasst werden.

EP 1 504 715 zeigt einen Katheter mit einem lichtemittierenden und einen lichtempfangenden Lichtleiter, deren Enden in einem vorbestimmten Abstand zueinander angeordnet sind.

Eine weitere Vorrichtung zur Messung des Blutflusses im Schädelinneren ist zum Beispiel aus der US 5,579,774 bekannt. Es wird eine Kathetersonde in das Innere des Gehirns eingeführt, die einen Messfühler zur Durchführung einer Laser-Doppler-Flowmetrie Messung umfasst. Über einen Lichtleiter wird der Lichtstrahl eines Helium-Neon Lasers mit 632,8 nm in axialer Richtung zu dem Messbereich geführt, der in distaler Richtung in Verlängerung der Sonde liegt. Das einfallende Licht wird zum Teil vom Umgebungsgewebe und zum Teil vom fliessenden Blut absorbiert und reflektiert. Das reflektierte Licht wird durch mindestens eine optische Faser zu einer Verarbeitungseinheit geführt. Das von den sich bewegenden roten Blutkörperchen reflektierte Licht unterliegt einer Doppelverschiebung, aus der die Durchflussrate ermittelt werden kann. Der Kopf der Kathetersonde weist eine abgerundete Spitze auf, deren Durchmesser sich konisch in distaler Richtung erweitert. Die abgestumpfte Spitze wird durch die Öffnung in der Schädeldecke und durch das darunter liegende Gewebe bis zu dem im Inneren des Hirns liegenden Messbereich vorgestossen. Dabei drückt die gesamte Fläche der stumpfen Spitze auf das zerebrale Gewebe und übt einen Druck aus, der bleibende Schäden im Hirn hinterlassen kann.

US 2006/0079813 offenbart eine Kathetersonde mit mehreren Teilstücken und mehreren Lumen. In den Teilstücken sind elektrische Sensoren angeordnet. Die Lumen dienen zur Zuführung von elektrischen Leitungen. Mehrere Teilstücke lassen sich nacheinander anordnen, so dass unterschiedliche Messungen durchgeführt werden können.

US 2004/0111016 zeigt einen Katheter mit einer Spitze, in welche Glasfasern geführt sind. In der Spitze ist ein Spiegel zur Umlenkung des Lichtstrahls angeordnet.

### Darstellung der Erfindung

Es ist eine Aufgabe der vorliegenden Erfindung, eine Vorrichtung zur Messung der Durchblutung in einem Körpergewebe, insbesondere in einem tiefliegenden zerebralen Gewebe, zu schaffen, welche sich einfach und kostengünstig herstellen lässt und die Messung nicht verfälscht.

Diese Aufgabe löst ein Katheter mit den Merkmalen des Anspruchs 1.

Es ist eine weitere Aufgabe der Erfindung, eine Vorrichtung zur Messung der Durchblutung in einem Körpergewebe, insbesondere in einem tiefliegenden zerebralen Gewebe, zu schaffen, die ein möglichst atraumatisches Einführen der Messvorrichtung in ein zerebrales Gewebe ermöglicht.

Diese Aufgabe löst ein Katheter mit den Merkmalen des Anspruchs 15.

Es ist eine weitere Aufgabe der Erfindung, eine Vorrichtung zur Messung der Durchblutung in einem Körpergewebe, insbesondere in einem tiefliegenden zerebralen Gewebe, zu schaffen, welche eine zuverlässige Messung des Durchflusses ermöglicht. Diese Aufgabe löst eine Vorrichtung mit den Merkmalen des Anspruchs 17.

In einer bevorzugten Ausführungsform des erfindungsgemässen Katheters ist eine einzige optische Faser als Lichtleiter für das zu emittierende Licht und das reflektierte Licht vorhanden, ein Photodetektor und ein Drainagekanal zur Drainage vorhanden.

In einer weiteren bevorzugten Ausführungsform des erfindungsgemässen Katheters ist eine einzige optische Faser als Lichtleiter für das zu emittierende Licht, ein Photodetektor im Katheter für das reflektierte Licht und ein Drainagekanal zur Drainage vorhanden.

In einer weiteren bevorzugten Ausführungsform des erfindungsgemässen Katheters ist das oben beschriebene Kopfteil, ein Drucksensor und ein Drainagekanal zur Drainage vorhanden.

Der erwähnte Drainagekanal kann zusätzlich oder alternativ als Führungskanal verwendet werden. Bei einem Katheter aus einem mehrheitlich flexiblen Material lässt sich ein steifer Draht durch den Führungskanal ziehen, welcher die Einführung des Katheters in das Körpergewebe erleichtert.

Eine bevorzugte Ausführungsform der Erfindung ist eine Vorrichtung zur Messung der Durchblutung in einem Körpergewebe einen Katheter mit einem Katheterkopf zum Einführen in das Innere eines Körpergewebes, einen optischen Leiter innerhalb des Katheters, eine Lichtquelle zur Emission eines Lichtstrahls in das Körpergewebe mittels dem optischen Leiter und eine Verarbeitungseinheit zur Ermittlung der Durchblutungsrate mittels einem in dem Körpergewebe reflektierten Lichtstrahls. Erfindungsgemäss weist der Katheter ein Mittelstück mit einer Ausnehmung, einer Einwölbung, einer Aussparung, einem Einschnitt oder einer Einbuchtung auf, die nach Innen in das Mittelstück ausgerichtet ist und seitlich relativ zur Längsachse des Katheters vorgesehen ist. Die Ausnehmung oder dergleichen kann dabei eine rundliche, ovalartige oder parabelartige Innenfläche aufweisen oder, wie z. B. im Falle eines Einschnitts aus mehreren Wänden zusammengesetzt sein. Die Ausnehmung oder dergleichen weist erfindungsgemäss einen Flächenbereich, aus dem der optische Leiter mündet und aus der somit der Lichtstrahl austritt, und einen weiteren Flächenbereich auf, der dem Flächenbereich, aus dem der optische Leiter mündet, gegenüberliegt und der zumindest teilweise schräg zur Achse des optischen Leiters und vorzugsweise auch schräg zur Längsachse des Katheters ausgerichtet ist. Die beiden Flächenbereiche werden im Folgenden aus Gründen der Einfachheit als Lichtaustrittsfläche, bzw. Lichtaustrittsflächenbereich, und als Reflexionsfläche, bzw. Reflexionsflächenbereich bezeichnet. Der optische Leiter tritt derart aus der Lichtaustrittsfläche aus, dass der aus der Lichtquelle emittierte Lichtstrahl auf die Reflexionsfläche gerichtet wird und an dieser in das umliegende Körpergewebe umgelenkt wird. Der Lichtstrahl wird in für die Durchblutung charakteristischer Weise im Körpergewebe absorbiert und reflektiert, wodurch sich ein Reflexionslichtstrahl bildet, der an der Reflexionsfläche reflektiert wird und in den einen optischen Leiter eingekoppelt wird. Vorzugsweise wird der Reflexionslichtstrahl an der Reflexionsfläche gebündelt. Der Reflexionslichtstrahl wird durch den optischen Leiter zur Verarbeitungseinheit übermittelt, wo sich aus diesem eingespeisten Signal im Vergleich mit dem Eingabesignal gemäss dem emittierten Lichtstrahl die Durchblutungsrate ermitteln lässt.

Der Katheter bildet somit eine Sonde bzw. Kathetersonde zur Messung der Durchblutung des Körpergewebes. Der Katheter weist einen besonders ausgebildeten Katheterkopf auf, wie weiter unten ausgeführt wird, wodurch er speziell für Messungen an zerebralem Gewebe, insbesondere an tiefliegendem zerebralem Gewebe, geeignet ist. Der optische Leiter kann z. B. durch ein Glasfaserkabel oder einen anderen Lichtleiter gegeben sein, der durch den Katheter bis zu dem Mittelstück und zu dessen Lichtaustrittsfläche geführt wird. Die Reflexionsfläche kann selbst eine Oberflächenrauhigkeit mit ausreichender Güte, vorzugsweise spiegelartiger Güte aufweisen, dass sie den Lichtstrahl aus dem Lichtleiter reflektieren kann. Vorzugsweise wird auf der schrägen Fläche ein Reflektor, wie etwa ein Spiegel angeordnet, um den Lichtstrahl in das umgebende Körpergewebe zu reflektieren. Ferner ist es vorteilhaft, wenn der Reflexionsflächenbereich gewölbt ausgebildet ist, um zum Einen den einfallenden Lichtstrahl fokussiert in das Gewebe reflektieren zu können und zum Anderen das am Gewebe reflektierte Licht in den optischen Leiter einspeisen zu können. Bei der Ausgestaltung der Ausnehmung mit ihren zugehörigen Flächenbereichen ist der Abstand vom Austritt des emittierten Lichtstrahls aus dem optischen Leiter bis zur gegenüberliegenden Reflexionsfläche bekannt und ist derart vorgesehen, dass der optische Leiter den emittierten Strahl auf den Brennpunkt der Reflexionsfläche fokussiert, sofern diese gewölbt ausgebildet ist. Vorzugsweise erfolgt eine Reflexion des emittierten Lichtstrahls in einem Winkel von 45° in das umliegende Gewebe. Es sind jedoch auch andere Reflexionswinkel denkbar, z. B. in einem Bereich von 30 ° - 60 °.

Die Ausnehmung ist vorzugsweise mit einem lichtdurchlässigen Material ausgefüllt, bzw. verschlossen oder versiegelt. Bevorzugt schliesst die äussere Fläche des Füllmaterials mit der Umfangsfläche des umliegenden Bereichs ab, so dass ein glatter Übergang entsteht. Als Füllmaterial kann z. B. Epoxidharz verwendet werden. Durch das Ausfüllen der Ausnehmung können Lufteinschlüsse im Strahlengang verhindert werden.

Es wird bevorzugt eine Lichtquelle mit kohärentem Licht verwendet, wie z. B. ein Laser oder eine Laserdiode, welche Licht im infrarotnahen Bereich zwischen 780 und 910 nm aussenden können. Licht in diesem Wellenlängenbereich ist in der Lage biologisches Gewebe zu penetrieren. Vorzugsweise werden gezielt Wellenlängen verwendet, die für ein ausgewähltes Messverfahren geeignet sind. Vorzugsweise wird Licht mit den Wellenlängen 785 nm, 850 nm und 905 nm verwendet, welches insbesondere von oxygeniertem und desoxygeniertem Hämoglobin und der Markersubstanz IndocyaninGrün absorbiert und reflektiert wird. Weiter ist es vorteilhaft einen getakteten Lichtstrahl oder auch getaktete Lichtstrahlen mit variabler Frequenz zu verwenden. Die an dem Gewebe reflektierten Anteile des einfallenden Lichtstrahls werden in der Verarbeitungseinheit mittels einem analog-digital-Umsetzer in ein aussagefähiges Signal bezüglich des zeitlichen Verlaufs der Anwesenheit von oxygenierten und desoxygeniertem Blut sowie der Markersubstanz transformiert.

Es kann jedoch auch eine Lichtquelle verwendet werden, die Licht über ein breites Wellenlängenspektrum aussendet, wie etwa eine Weisslichtquelle. Die für die Messung des Blutdurchflusses relevanten Wellenlängenbereiche können dann durch ein Spektrometer erfasst werden. Kohärente Lichtquellen haben jedoch den Vorteil, dass sie einen geringeren Energiebedarf haben.

Die Kathetersonde ist auch für weit im Inneren des Gehirns liegende Gewebebereiche geeignet. Vorteilhafterweise ist hierfür der Abstand zwischen dem Katheterkopf und dem Mittelstück variabel. Je nach gewünschtem Messbereich, bzw. gewünschter Eindringtiefe in das Gehirn, kann ein mehr oder weniger langes Verbindungselement, z. B. in Form eines Schlauches verwendet werden. Der Katheterkopf kann z. B. bis zum Ventrikelboden in das Gehirn eingeführt werden, der dann u. a. als Referenzpunkt für die Lokalisierung des Mittelstücks dienen kann. Die Messung des Blutdurchflusses kann somit unmittelbar vor Ort in den interessierenden Bereichen des Körpergewebes durchgeführt werden.

Vorzugsweise umfasst die Vorrichtung zur Messung der Durchblutung in einem Körpergewebe gemäss der Erfindung auch eine Halteeinrichtung, die auf der Kopfoberfläche über einer Einführöffnung in der Schädeldecke angeordnet wird und die die Kathetersonde in einer vorbestimmten Position hält. Die Halteeinrichtung weist z. B. eine Auflagefläche zum Aufsetzen auf die Kopfoberfläche und davon abragend ein Führungsrohr für den Katheter auf. Mit der Halteeinrichtung kann das Eindringen von infektiösen Substanzen in das Gewebe während einer Messung verhindert werden. Sie wirkt als Barriere gegen Krankheitserreger und Verunreinigungen. Vorzugsweise ist das Führungsrohr senkrecht auf der Auflagefläche angeordnet, so dass es den Katheterkopf senkrecht durch die Schädeldecke führt und anschliessend senkrecht in Position hält. In dieser Position kann die Kathetersonde um ihre Achse gedreht werden, ohne aus ihrer Position ausgelenkt zu werden und umliegendes Gewebe zu schädigen. Durch das Drehen der Sonde wird das erreichbare Messfeld deutlich erweitert. Es kann eine Messung in einem 360° Sektor um das Mittelstück der Kathetersonde erfasst werden.

Weiter kann die Kathetersonde wenigstens einen Röntgenmarker aufweisen, der bei der Durchführung einer Röntgenmessung die Position und die Ausrichtung der Sonde im Gewebe angeben kann.

Sind Katheterkopf und Mittelstück aus Metall gefertigt, kann ihre Position durch Röntgenverfahren überwacht werden. Grundsätzlich sind jedoch auch andere körperverträgliche Materialien denkbar, wobei in diesem Fall die Überwachung durch Röntgenverfahren durch opale Zusatzstoffe, durch bereits im Katheter verwendete Elektronikbauteile oder durch Marker erreichbar ist. Ferner kann der Katheter mit einer Skala für die Eindringtiefe in das Körpergewebe und auch mit einer Winkelskala zur Angabe der Winkelstellung des Mittelstücks versehen sein. Durch diese Massnahmen ist eine exakte Lokalisierung des Messbereichs im Gehirn möglich.

Gleichzeitig kann die Kathetersonde mit zusätzlichen Messfühlern ausgestattet werden. Beispielsweise kann in dem Mittelstück ein Temperaturmessfühler, wie etwa ein Thermistor oder ein Thermokuppler, vorgesehen werden. Der Temperaturfühler kann in einem Aussenbereich des Mittelstücks z. B. in einem Kanal angeordnet werden. Da Metall einen ausgezeichneten Wärmeleitfähigkeitskoeffizienten aufweist, ist gleichzeitig mit der Messung des Blutflusses in dem Umgebungsgewebe des Mittelstücks auch eine Temperaturerfassung des Gewebes möglich. Ein Kontakt mit dem Gewebe ist in diesem Fall nicht erforderlich. Sofern aber das Mittelstück aus Kunststoff hergestellt ist, sollte ein Kontakt zwischen Messfühler und Gewebe gewährleistet werden. Die für die Messung erforderlichen Leitungen können durch den Katheter zur Verarbeitungseinheit geführt werden, in der das Temperatursignal aufgenommen und umgesetzt wird. Es ist aber auch möglich, einen Temperaturmessfühler in einem Schlauchbereich zwischen der Spitze der Sonde und dem Mittelstück anzuordnen.

Ferner können der Katheterkopf und das Mittelstück des Katheters mit einer Durchführung, bzw. einem Drainagekanal versehen sein, der eine Drainage des umliegenden Gewebes durch den Katheter ermöglicht. Hierfür weist z. B. der Katheterkopf wenigstens eine Öffnung zum Umgebungsgewebe auf, die mit dem Drainagekanal verbunden ist, so dass Flüssigkeit aus dem Gewebe durch den Kanal aspiriert werden kann. Bevorzugt ist nahe der Drainageöffnung zumindest ein Drucksensor vorgesehen, der zum Einen zur Bestimmung des Drucks in dem Umgebungsgewebe und zum anderen zur Bestimmung des Drucks in dem Drainagekanal geeignet ist. Bei einer Verlegung oder Okklusion und einer folgenden Druckänderung im Drainagekanal kann ein Signal an die Verarbeitungseinheit abgegeben werden, so dass ein Alarm ausgelöst werden kann. Grundsätzlich ist es auch möglich über die Drainageöffung dem Gewebe Flüssigkeit zu zu führen, d. h. Flüssigkeit zu injizieren.

Nach einem weiteren Aspekt der vorliegenden Erfindung ist ein Katheterkopf zum Einführen in ein Körpergewebe vorgesehen, der sich in einen Einführbereich und einen sich daran anschliessenden Anschlussbereich unterteilt. Der Einführbereich umfasst in seiner Oberfläche mehrere Aussparungen. Ferner weist der Einführbereich einen in Richtung des Anschlussbereichs zunehmenden Durchmesser auf. Die Aussparungen sind derart im Einführbereich vorgesehen, dass zwischen den Aussparungen entlang der Oberfläche des Katheterkopfes Stege in Richtung des Anschlussbereichs verlaufend ausgebildet werden. Die Stege beginnen in einem Bereich des Einführbereichs mit geringem Durchmesser und enden in einem Bereich mit grösserem Durchmesser.

Durch diese erfindungsgemässe Ausgestaltung des Katheterkopfes wird ein sanftes, möglichst atraumatisches Einführen des Katheterkopfes in und durch das Körpergewebe, insbesondere ein zerebrales Gewebe möglich. Beim Einführen des Katheterkopfes wird das Gewebe zunächst nur durch den vordersten Bereich und die Oberfläche der Stege aufgespreizt. Im Bereich der Aussparungen zwischen den Stegen wird das Gewebe noch nicht belastet. Nachdem diese erste Aufdehnung erfolgt ist, wird der Katheterkopf weiter eingeführt und auch die Gewebebereiche an den Aussparungen werden durch die Umfangsfläche des Anschlussbereichs aufgeweitet. Bei diesem Vorgehen wird nur ein minimaler direkter Druck auf das Gewebe ausgeübt.

Der Anschlussbereich des Katheterkopfes ist vorzugweise kreisförmig oder auch oval ausgebildet und weist einen einheitlichen Durchmesser auf. Der Durchmesser beträgt bevorzugt maximal 3mm. Der Einführbereich ist im vordersten Bereich der Spitze geschlossen und ist z. B. parabelförmig, rund oder als stumpfer Kegel ausgebildet. Die Aussparungen können sich von dem Einführbereich bis zu dem Anschlussbereich in länglicher Form erstrecken. Die Stege zwischen den Aussparungen sind auf Grund des zunehmenden Durchmessers des Einführbereichs in Längsrichtung des Katheters gewölbt ausgebildet. In einer Draufsicht auf die Spitze ergibt sich daraus eine kreuzartige oder sternförmige Anordnung der Stege, je nach Anzahl der Aussparungen.

In einer Ausführungsform der Erfindung weisen die Aussparungen Öffnungen auf, vorzugsweise werden sie vollständig durch Öffnungen gebildet. Die Ränder oder Kanten der Aussparungen bzw. Öffnungen sind abgerundet oder abgeschrägt, so dass sich ein gleitender Übergang der angrenzenden Flächen ergibt. Der Katheterkopf kann im Inneren einen Kanal aufweisen, der mit einem Kanal des Katheters eine Ableitung in proximaler Richtung bilden kann. Der Kanal ist mit den Öffnungen verbunden, welche die Aussparungen bilden. Es ist somit möglich, die Öffnungen als Drainageöffnungen zur Ableitung oder Zuführung von Flüssigkeit aus dem Umgebungsgewebe zu nutzen. Um den Drainagekanal zu bilden kann sich an den Anschlussbereich in Längsrichtung ein Befestigungsstück anschliessen, das zur Befestigung eines Verbindungsrohrs, vorzugsweise eines flexiblen Schlauchteils, des Katheters dient. Dabei entspricht der Durchmesser des Verbindungsrohrs bzw. des Schlauchteils auf dem Befestigungsstück im Wesentlichen dem Durchmesser des Anschlussbereichs, so dass sich ein glatter Übergang zwischen diesen Bauteilen ergibt.

### Kurze Beschreibung der Zeichnungen

In den Zeichnungen ist eine Ausführungsform gemäss der vorliegenden Erfindung gezeigt. An den Figuren offenbar werdende Merkmale der Messvorrichtung sollen als zum Umfang der Offenbarung gehörend angesehen werden und in keiner Weise einschränkend verstanden werden. In den Zeichnungen stellen dar:
- Figur 1:: Gesamtansicht einer Messvorrichtung gemäss der vorliegenden Erfindung;
- Figur 2:: dreidimensionale Darstellung eines Katheterkopfes nach der vorliegenden Erfindung;
- Figur 3:: Frontansicht des Katheterkopfes nach Figur 2;
- Figur 4:: Längsschnitt durch den Katheterkopf nach Figur 3; und
- Figur 5:: dreidimensionale Darstellung einer ersten Ausführungsform eines Mittelstücks einer Messvorrichtung gemäss der vorliegenden Erfindung;
- Figur 6a:: Längsschnitt durch eine Messvorrichtung nach Figur 1 entlang einer ersten Längsebene;
- Figur 6b:: Längsschnitt durch eine Messvorrichtung nach Figur 1 entlang einer zweiten Längsebene, senkrecht zur ersten Ebene;
- Figur 7:: dreidimensionale Darstellung einer zweiten Ausführungsform eines Mittelstücks einer Messvorrichtung gemäss der vorliegenden Erfindung;
- Figur 8:: Längsschnitt durch eine Messvorrichtung nach Figur 1 gemäss der zweiten Ausführungsform;
- Figur 9: eine perspektivische Darstellung eines erfindungsgemässen Mittelstücks;
- Figur 10: eine perspektivische Darstellung eines Deckels des Mittelstücks gemäss Figur 9;
- Figur 11: eine Ansicht des distalen Endes eines Unterteils gemäss Figur 9;
- Figur 12: eine Ansicht des proximalen Endes des Unterteils des Mittelstücks gemäss Figur 9;
- Figur 13: eine perspektivische Darstellung des Unterteils gemäss Figur 12;
- Figur 14: eine Ansicht von oben des Unterteils gemäss Figur 12;
- Figur 15: einen Längsschnitt durch das Unterteil gemäss Figur 12 mit Lichtleiter und Platine;
- Figur 16: eine schematische Darstellung der erfindungsgemässen Messvorrichtung und
- Figur 17: dreidimensionale Darstellung eines Katheterkopfes nach der vorliegenden Erfindung in einer weiteren Ausführungsform.

### Ausführliche Beschreibung_bevorzugter Ausführungsformen

Im Folgenden wird als distales Ende einer Kathetersonde das Ende mit der Katheterspitze und als proximales Ende das gegenüberliegende Ende bezeichnet.

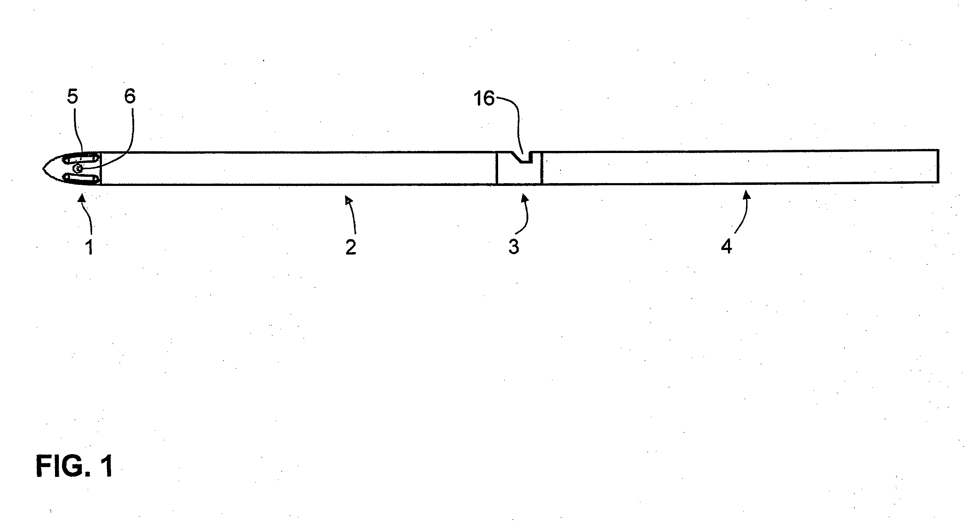

In Figur 1 ist eine Messvorrichtung gemäss der vorliegenden Erfindung gezeigt, die als Katheterkopf ein Spitzenelement 1, einen ersten Verbindungsrohr 2, ein Mittelstück 3 und ein zweites Verbindungsrohr 4 aufweist. Das erste und zweite Verbindungsrohr 2, 4 sind vorzugsweise je aus einem flexiblen Schlauchteil gebildet. Im folgenden wird deshalb von Schlauchteil gesprochen, obwohl auch andere Arten von Verbindungsrohren 2, 4, welche eine Einführung des Katheters in das Körpergewebe erlauben, darunter verstanden werden.

Das Spitzenelement 1 ist am distalen Ende der Messvorrichtung vorgesehen. Am proximalen Ende weist die Messvorrichtung eine Steckerverbindung auf (nicht dargestellt) oder wird direkt in eine Verarbeitungseinheit zur Umsetzung und Auswertung der Messsignale geführt. Das Spitzenelement weist mehrere längliche Öffnungen 5 auf, die Aussparungen in der Oberfläche des Katheterkopfes bilden. Die Öffnungen 5 sind zur Drainage von Flüssigkeit im umliegenden Gewebe vorgesehen. Ferner ist eine runde Öffnung 6 zwischen zwei länglichen Öffnungen 5 vorgesehen, in der ein Drucksensor angeordnet werden kann. Der Drucksensor kann jedoch auch benachbart, jedoch nicht zwischen diesen Spülöffnungen 5 angeordnet sein. Als Drucksensoren können beispielsweise elektronische oder optomechanische Einrichtungen, wie etwa eine Siliziummikromembran, verwendet werden. Das Mittelstück 3 weist einen Einschnitt, bzw. eine Aussparung 16 auf, in die ein Lichtleiter mündet und durch die durch den Lichtleiter zugeführtes Licht in das Umgebungsmedium emittiert werden kann. Der erste Schlauchteil 2 kann in seiner Länge je nach vorgesehener Anwendungsart der Messvorrichtung variiert werden.

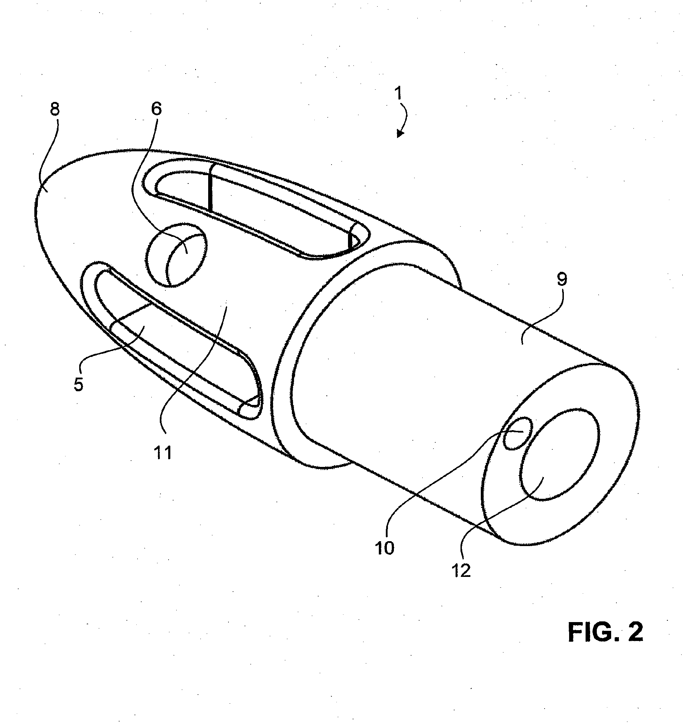

In Figur 2 ist der Katheterkopf in Form des Spitzenelements 1 in einer dreidimensionalen Darstellung gezeigt. Das Spitzenelement kann aus Kunststoff oder aus Metall gefertigt sein. Das Spitzenelement unterteilt sich in einen distalen Einführbereich und einen sich daran anschliessenden Anschlussbereich. Es weist im Einführbereich eine ovale, parabelförmige oder rundliche Spitze 8 auf. Von der Spitze 8 an weist der Einführbereich in Richtung des Anschlussbereichs einen zunehmenden Durchmesser auf. Die Spitze 8 ist in ihrem Zentrum geschlossen. Kurz danach schliessen sich in Umfangsrichtung nebeneinander angeordnet eine oder mehrere längliche Aussparungen, bzw. Öffnungen 5 an. In der in Figur 2 gezeigten Ausführungsform sind vier Öffnungen vorgesehen, wovon zwei in Figur 2 ersichtlich sind. Zwischen den länglichen Öffnungen 5 bilden sich Stege 11 im Umfangsbereich aus, die sich vom Anschlussbereich bis in den zusammenlaufenden Bereich der Spitze 8 erstrecken und somit leicht gewölbt sind. Die Randbereiche der länglichen Öffnungen 5 sind abgerundet bzw. entgratet, so dass ein sanfter Übergang von der Umfangsfläche des Katheterkopfes zu den Randbereichen der Öffnungen 5 entsteht. Durch die leichte Wölbung der Stege laufen diese im Spitzenbereich auf das Zentrum, bzw. in radialer Richtung aufeinander zu. Der Zwischenraum zwischen zwei benachbarten länglichen Öffnungen 5, der die Stege 11 bildet, ist annähernd so breit, wie die Breite einer länglichen Öffnung. Durch die leichte Wölbung der Stege verjüngt sich dieser Umfangsbereich in Richtung der Spitze.

Am proximalen Ende weist das Spitzenelement 1 ein Befestigungsstück 9 auf, an welches sich der erste Schlauchteil 2 anschliesst. Das Befestigungsstück 9 ist Teil des Anschlussbereichs, bzw. schliesst sich an diesen an. Das Befestigungsstück 9 ist hülsenartig ausgebildet. Über den Umfang des Befestigungsstücks 9 kann ein Schlauchgebilde formschlüssig aufgesetzt werden. In der Umfangswand des Befestigungsstücks 9 verläuft in Längsrichtung ein Kanal 10 durchgehend vom distalen Ende bis in den Einführbereich. Der Kanal 10 dient zur Durchleitung der Elemente einer Druckmessung. Beispielsweise können elektrische oder optische Leitungen durch den Kanal 10 bis zur Öffnung 6 geführt werden, in der ein Drucksensor angeordnet werden kann.

Die länglichen Öffnungen 5 dienen zur Drainage des Umgebungsgewebes. Die Drainageflüssigkeit wird durch einen axial verlaufenden Kanal 12 abgeführt, der sich bis in den Einführbereich des Katheterkopfes erstreckt. Es ist ein Vorteil der vorliegenden Erfindung, dass der Drucksensor zwischen den länglichen Öffnungen 5 angeordnet werden kann und somit auf der gleichen Höhe im Umgebungsgewebe eine Druckmessung durchführt, wie auch eine Flüssigkeit durch die Drainageöffnung abgeführt werden kann.

Sofern die Spitze und das Mittelstück aus Kunststoff gefertigt ist, weist die Spitze vorzugsweise eine Röntgenmarkierung auf, um die Positionierung der Spitze überprüfen zu können.

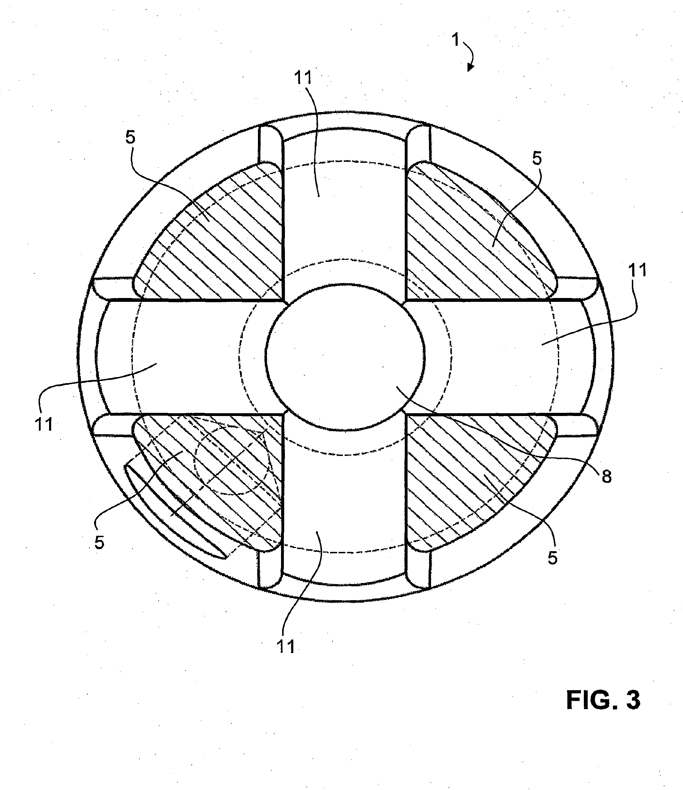

In Figur 3 ist eine schematische Darstellung einer Frontansicht eines Spitzenelements 1 gezeigt. Im Zentrum ist die Spitze 8 als vorderste Wölbung des Katheterkopfes zu sehen. Um dieses Zentrum herum sind die vier Aussparungen in Form von länglichen Öffnungen 5 angeordnet, die in der Draufsicht einen freien Raum zwischen sich ausbilden. Die Umfangsbereiche zwischen den länglichen Öffnungen 5 bilden die Stege 11 aus, die vom Zentrum der Spitze 8 bis zum Aussenumfang des Anschlussbereichs verlaufen und eine Art Führungsstruktur für das Spitzenelement 1 beim Einführen in ein zerebrales Gewebe bilden. In der Draufsicht in Figur 3 erscheinen die Stege 11 in einer kreuzartigen Anordnung. Wird der Katheterkopf in das Gewebe eingeführt, wird die Spitze 8, deren Durchmesser kleiner ist als der Durchmesser des gesamten Spitzenelements, und die Fläche der Stege 11 unmittelbar auf das Gewebe gedrückt. In dem Bereich der Aussparungen, das heisst im Bereich der länglichen Öffnungen 5, wird zunächst kein Druck auf das Gewebe ausgeübt. Durch die Stege 11 wird das Gewebe vorsichtig aufgespannt und auseinandergespreizt, so dass der Katheterkopf in das Gewebe eindringen kann, und dabei möglichst wenig Traumatisierung hervorruft. Erst nachdem eine erste Aufweitung des Gewebes im Einführbereich des Spitzenelements 1 durch die Stege 11 erfolgt ist, wird das Gewebe vollständig über den gesamten Durchmesser des Anschlussbereichs aufgespreizt. Durch diese Ausgestaltung des Katheterkopfes der Messvorrichtung ist es möglich, die Messvorrichtung nahezu verletzungsfrei in innere Bereiche des Gehirns einzuführen.

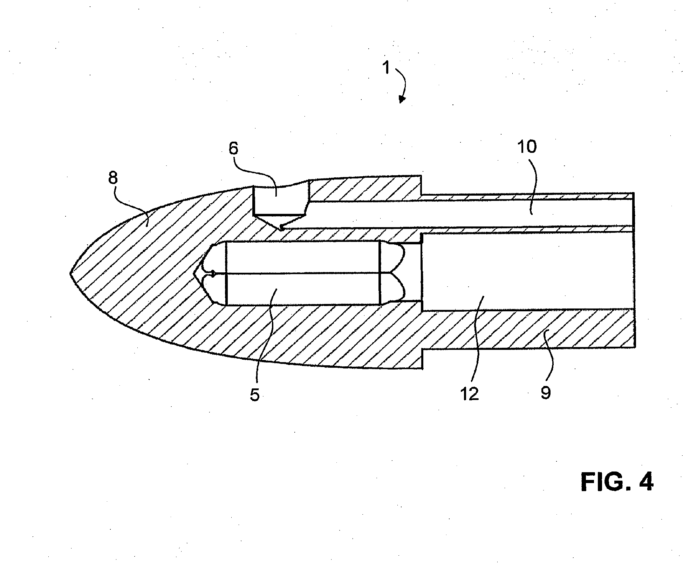

In Figur 4 ist eine Schnittzeichnung entlang der Längsachse eines Spitzenelements 1 dargestellt. Es ist deutlich ersichtlich, dass das Spitzenelement 1 im Einführbereich und der Spitze 8 einen geringeren Durchmesser aufweist, der in proximaler Richtung zunimmt bis zum Anschlussbereich, an den sich das Befestigungsstück 9 anschliesst. Der Kanal 10 verläuft geradlinig in Längsrichtung bis zur Öffnung 6, die für einen Drucksensor vorgesehen ist. Im Inneren schliesst sich an die vier länglichen Öffnungen 5 ein Drainagekanal 12 an, der zur Ableitung von Drainageflüssigkeit vorgesehen ist. Der vorderste Bereich der Spitze 8 ist in dieser Darstellung derart spitz dargestellt, dass die Aufweitung des Gewebes optimiert wird, jedoch keine Verletzungen durch ein Ritzen oder Schneiden entstehen können. Im Wesentlichen ist die Spitze 8, bzw. der Querschnitt durch den Katheterkopf durch die Stege 11 parabelförmig.

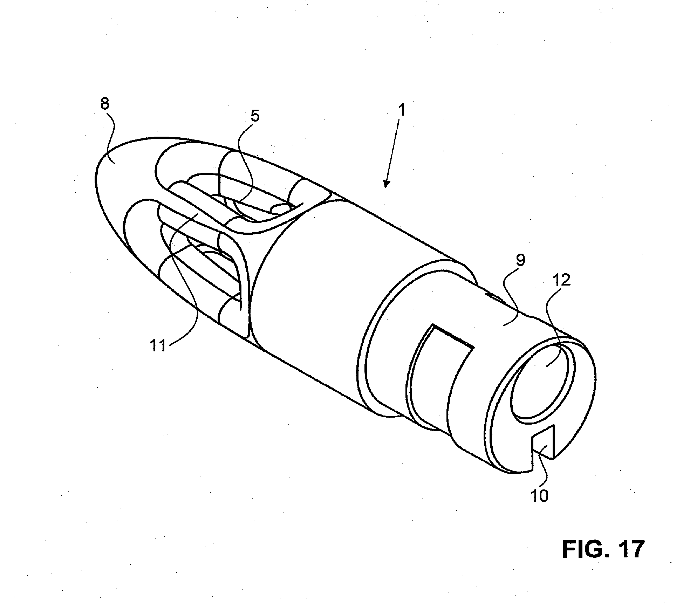

Figur 17 zeigt eine zu Figur 2 alternative Ausführungsform eines Spitzenelements 1. Gleiche Teile sind mit gleichen Bezugszeichen versehen. Der Spitze 8 ist wiederum gerundet ausgebildet. Die Öffnungen 5 liegen näher beieinander als in der Ausführungsform gemäss der Figur 2 und sie sind lediglich durch schmale Stege voneinander getrennt. Die Stege weisen vorzugsweise eine Breite auf, welche um ein Vielfaches kleiner als die Breite der einzelnen Öffnungen ist. Der Drainagekanal 12 verläuft axial versetzt zur Längsmittelachse des Spitzenteils 1. Der Drucksensorkanal bzw. der Kanal für andere elektrische Leitungen 10 ist hier offen ausgebildet. Der Drucksensor ist vorzugsweise auf der der Spitze 8 gegenüberliegenden Seite der Öffnungen 5 angeordnet oder er befindet sich im Mittelstück.

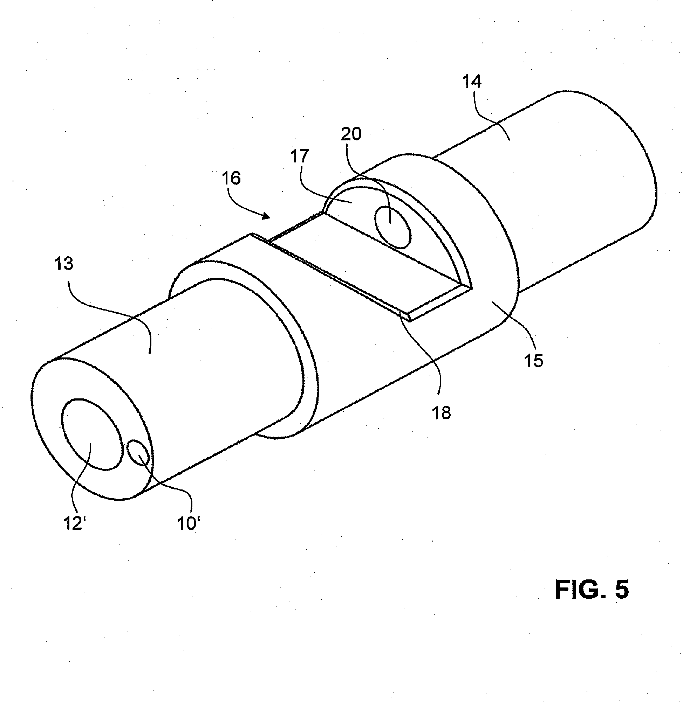

In Figur 5 ist ein Mittelstück 3 gemäss der vorliegenden Erfindung gezeigt. Das Mittelstück 3 weist ein distales Befestigungsstück 13 und ein proximales Befestigungsstück 14 auf. Die Befestigungsstücke 13 und 14 sind dem Befestigungsstück 9 ähnlich. Sie sind hülsenartig ausgebildet und weisen einen zentralen Durchgang für einen Drainagekanal 12' zur Drainage von Flüssigkeit und in ihrer Umfangswand einen durchgehenden Kanal 10 für Leitungen für den Drucksensor im Spitzenelement 1 auf. Der Führungs-, bzw. Drainagekanal 12' im Mittelstück 3 ist analog dem Kanal 12 im Spitzenelement 1 vorgesehen und führt diesen fort.

Zwischen dem proximalen Befestigungsstück 13 und dem proximalen Befestigungsstück 14 ist an dem Mittelstück 3 ein Mittelbereich 15 ausgebildet. Der Mittelbereich 15 weist in der Ausführungsform gemäss Figur 5 einen keilartigen Einschnitt 16 auf, der jedoch nicht bis zum Drainagekanal 12' hindurchreicht, sondern diesen geschlossen lässt. Der Einschnitt 16 verläuft an seinem proximalen Ende mit einer Lichtaustrittsfläche 17, aus der ein optischer Leiter mündet, senkrecht zur Längsachse. An seinem distalen Ende weist der Einschnitt 16 eine Reflexionsfläche 18 auf, die vorzugsweise in einem 45°-Winkel zur Längsachse des Mittelstücks 3 und zur Fläche 17 verläuft. Durch den Umfang des distalen Befestigungsstücks 14 und das proximale Ende des Mittelstücks 3 verläuft in Längsrichtung des Mittelstücks 3 ein Kanal 20, der in der Lichtaustrittsfläche 17 des Einschnitts 16 endet. Die Öffnung des Kanals 20 im Einschnitt 16 liegt somit der schrägen Fläche 18 gegenüber. Der Kanal 20 ist zur Führung eines Lichtleiters vorgesehen. Der Lichtleiter kann auch über die Lichtaustrittsfläche überstehen und in den Einschnitt 16 hineinragen. Das Licht aus dem Lichtleiter trifft auf die gegenüberliegende Reflexionsfläche 18. Auf der Reflexionsfläche 18 ist ein Spiegel oder sonstiger Reflektor angeordnet, der das Licht aus dem Lichtleiter in das Umgebungsgewebe um das Mittelstück 3 reflektiert. Der Reflektor kann als gesondertes Element auf der Reflexionsfläche 18 angebracht werden oder die Reflexionsfläche 18 selbst kann als Reflektorfläche ausgearbeitet sein. Beispielsweise kann eine Goldbeschichtung als Reflexionsfläche vorgesehen sein. Es ist auch möglich, die Fläche 18, bzw. den Reflektor, leicht gewölbt auszubilden, so dass der auftreffende Lichtstrahl leicht aufgeweitet wird. Der Einschnitt 16 ist mit Epoxidharz gefüllt, so dass die Oberfläche des Mittelbereichs 15 zylindrisch ausgebildet ist.

Als Lichtleiter eignet sich bevorzugt eine einzelne optische Faser. Vorzugsweise sind nicht mehr als fünf optische Fasern vorhanden. Zwei oder drei Fasern können ebenfalls verwendet werden.

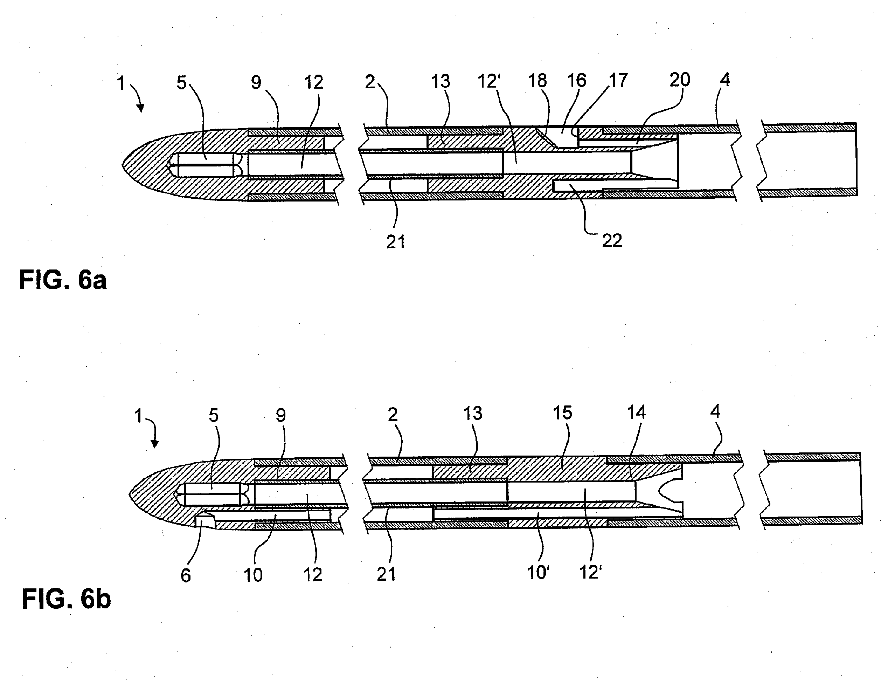

In den Figuren 6a und 6b sind jeweils Längsschnitte einer ersten Ausführungsform einer Kathertersonde gemäss der vorliegenden Erfindung gezeigt, die zueinander um 90° versetzt sind. In Figur 6a ist von links nach rechts ein Spitzenelement 1, erster Schlauchteil 2, eine Mittelstück 3 und ein zweiter Schlauchteil 4 gemäss der Darstellung aus Figur 1 gezeigt. Der erste Schlauchteil 2 ist mit einem Ende über das Befestigungsstück 9 des Spitzenelements 1 und mit dem anderen Ende über das Befestigungsstück 13 des Mittelstücks 3 gestülpt. Im Inneren des ersten Schlauchteils ist ein weiterer flexibler Schlauch 21 zwischen dem Spitzenelement 1 und dem Mittelstück 3 vorgesehen, welcher den die Führungskanäle 12 und 12' des Spitzenelements und des Mittelstücks mit einander verbindet. Die Kanäle 12 und 12' bilden gemeinsam mit dem Schlauch 21 einen Drainagekanal für Flüssigkeit, die aus dem Umgebungsgewebe der Katheterspitze durch die Öffnungen 5 abgeleitet werden soll. Die Schlauchteile 4 und 21 können in ihrer Länge variiert werden, so dass auch die Position des Mittelteils 3 im Gewebe angepasst werden kann, d. h. es können verschieden lange Schlauchteile zwischen dem Spitzenelement und dem Mittelstück eingesetzt werden.

Am Mittelstück 3 ist der keilartige Einschnitt 16 mit der Lichtaustrittsfläche 17 und der Reflexionsfläche 18 ersichtlich. Der Kanal 20 mündet in die Lichtaustrittsfläche 17. Dabei verläuft die Lichtaustrittsfläche 17 im Wesentlichen senkrecht zur Längsachse des Katheters, könnte jedoch auch gewinkelt dazu vorgesehen sein. In dem Kanal 20 wird ein optischer Leiter geführt, der weiter durch den zweiten Schlauchteil 4 zu einer Lichtquelle geführt ist (nicht dargestellt).

Weiter ist in der Umfangswand des Mittelstücks 3 auf der proximalen Seite ein Temperaturmesskanal 22 vorgesehen, der auf der Seite des zweiten Schlauchteils 4 beginnt und ca. in der Mitte des Mittelstücks 3 endet. Der Temperaturmesskanal 22 ist für einen Temperaturfühler vorgesehen, welcher die Temperatur des Umgebungsgewebes misst. Der Temperaturfühler ist an dieser Stelle nahe am Aussenumfang des Katheters angeordnet und kann ohne Beeinträchtigung oder Verfälschung die Temperatur im Gewebe messen. Von dem Temperaturfühler verlaufen Leitungen (nicht dargestellt) durch den zweiten Schlauchteil 4 zu der Verarbeitungseinheit und übermitteln ein Temperatursignal an die Verarbeitungseinheit.

In Figur 6b ist im Spitzenelement 1 der Drucksensorkanal 10 ersichtlich, der sich vom proximalen Ende des Spitzenelements 1 bis zur Öffnung 6 erstreckt. In der Öffnung 6 ist ein Drucksensor vorgesehen, dessen Leitungen durch den Kanal 10 und den Zwischenraum zwischen dem ersten Schlauchteil 2 und dem Innenschlauch 21 bis zu einem Kanal 10' im Mittelstück 3 verlaufen. Am proximalen Ende des Mittelstücks 3 treten die Leitungen aus dem Kanal 10' aus und werden weiter durch den zweiten Schlauchteil 4 bis zur Verarbeitungseinheit geführt, der sie ein Signal entsprechend dem Druck im Umgebungsmedium des Spitzenelements 1 oder im Drainagekanal 12 übermitteln. In Abhängigkeit des Drucksignals des Drucksensors kann die Verarbeitungseinheit die Drainage von Flüssigkeit durch den Drainagekanal steuern.

Ebenso kann auch eine manuelle Drainage durchgeführt werden.

Der Kanal 12' am Mittelstück 3 ist an seinem distalen Ende konisch aufgeweitet. Diese trichterförmige Öffnung erleichtert das Einführen eines Führungsdrahtes, der zur Führung der Kathetersonde beim Einführen in das Körpergewebe verwendet wird.

Im Inneren des zweiten Schlauchteils 4 können die Leitungen für den Drucksensor und den Temperaturfühler und der optische Lichtleiter frei geführt werden. Es kann jedoch auch in diesem Schlauchteil ein Innenschlauch vorgesehen werden, so dass die Leitungen im Zwischenraum zwischen dem äusseren und dem inneren Schlauch geführt werden. An den zweiten Schlauchteil 4 kann sich ein Stecker anschliessen, in den die Leitungen enden. Der Stecker kann direkt an die Verarbeitungseinheit oder an einen weiteren Stecker angeschlossen, der wiederum zu der Verarbeitungseinheit führt.

Bei der Untersuchung eines Körpergewebes, wie etwa des zerebralen Gewebes im Inneren des Gehirns, wird eine Öffnung in der Schädeldecke vorgesehen. Über der Öffnung wird eine Halteeinrichtung für den Katheter vorgesehen, welche den Katheter beim Einführen und in eingeführtem Zustand möglichst senkrecht zur Kopfoberfläche hält. Die Halteeinrichtung deckt somit zum einen die Öffnung im Kopf ab und sorgt zum anderen dafür, das der Katheter beim Einführen und Messen nicht unerwünscht aus der vorgesehenen Bahn ausgelenkt wird. Dadurch kann eine Verunreinigung des Untersuchungsbereichs und eine unnötige Verletzung des umliegenden Gewebes verhindert werden.

Die Kathetersonde wird vorsichtig durch das Gewebe bis zu einer gewünschten Messposition eingeführt, beispielsweise bis sie auf einem Ventrikelboden aufsteht. Durch die erfindungsgemässe Ausgestaltung des Katheterkopfes wird dabei nur minimaler Einfluss auf das zerebrale Gewebe ausgeübt, so dass das Gewebe nicht unnötig geschädigt und die Messung nicht verfälscht wird.

Zur Messung der Durchblutung des zerebralen Gewebes wird Licht im nahen Infrarotbereich durch den Lichtleiter im Kanal 20 bis auf die Reflexionsfläche 18 geleitet. Die Lichtquelle kann dabei. B. auch an der Verarbeitungseinheit vorgesehen sein. Der auf die Fläche 18 gelenkte Lichtstrahl wird dort reflektiert und in das zerebrale Gewebe ausgesendet. Dort wird es u. a. am oxigenierten und desoxigenierten Hämoglobin und ggf. am Indocyaningrün absorbiert und reflektiert. Das reflektierte Licht fällt teilweise auf die Reflexionsfläche 18 zurück, wodurch es durch die Reflexion an der Fläche 18 in den Lichtleiter im Kanal 20 eingespeist wird. Der Kanal führt das reflektierte Licht zur Verarbeitungseinheit zurück, in der es z. B. mittels eines Analog-/Digital-Wandlers ausgewertet wird. Gleichzeitig kann über den Temperaturfühler im Kanal 22 die Temperatur im gemessenen Gewebe und mit dem Drucksensor der Druck im Umfeld der Sondenspitze bestimmt werden. Falls erforderlich, kann sofort eine Drainage mit dem Kanal 12, 12' eingeleitet werden.

Durch die Führung der Messsonde mittel der Halteeinrichtung kann die Sonde in ihrer Messposition verdreht werden, ohne dabei ausgelenkt zu werden und das umliegende Gewebe zu schädigen. Durch die Drehung der Sonde kann der Messbereich im Gewebe deutlich ausgeweitet werden. Es ist möglich, ein Messfeld in einem 360° Umkreis um das Mittelstück 3 zu erfassen. Die Schlauchteile zwischen der Spitze und dem Mittelstück können dabei flexibel oder starr ausgebildet sein.

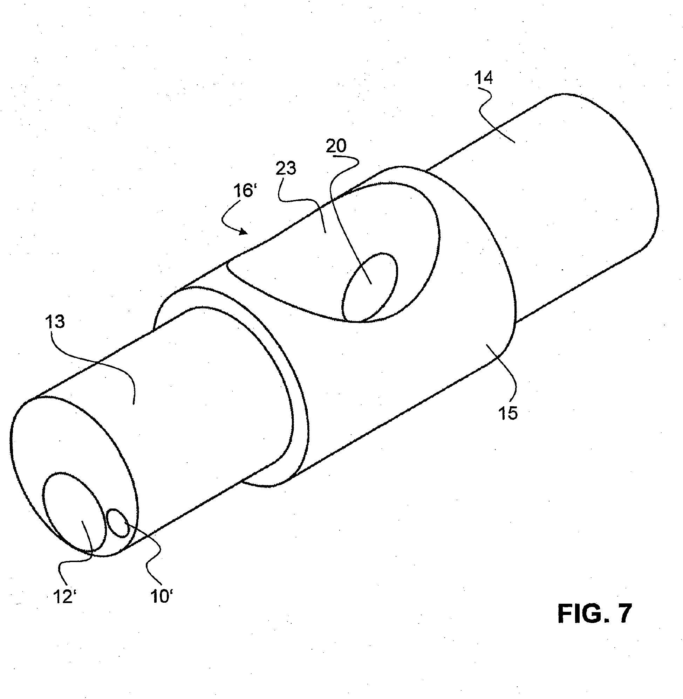

In Figur 7 ist eine zweite Ausführungsform eines Mittelstücks gemäss der vorliegenden Erfindung gezeigt. Bei dieser Ausgestaltung des Mittelstücks ist zum Einen der Drainagekanal 12' exzentrisch zur Längsachse angeordnet. Dadurch kann das Mittelstück mit einem geringeren Durchmesser gebaut werden. Zum Anderen ist als erfindungsgemässe Ausnehmung eine Aussparung 16' vorgesehen, die sich gewölbt, bzw. rundlich in das Mittelstück vertieft. Eine dadurch ausgebildete Innenfläche 23 umfasst am proximalen Bereich einen Lichtaustrittsflächenbereich und am distalen Bereich einen Reflexionsflächenbereich, der dem Lichtaustrittsflächenbereich gegenüberliegt. Der Lichtaustrittsflächenbereich entspricht in seiner Funktion der Lichtaustrittsfläche 17 aus der Ausführungsform gemäss den Figuren 1 bis 6 und der Reflexionsflächen bereich entspricht demgemäss in seiner Funktion der Reflexionsfläche 18. Der Lichtleiterkanal 20 mündet aus dem Lichtaustrittsflächenbereich. Licht, das aus einem optischen Leiter im Lichtleiterkanal 20 emittiert wird, trifft auf den gegenüberliegenden Reflexionsflächenbereich von dem es in das umgeliegende Gewebe reflektiert wird. Die Wölbung der Aussparung 16' ist derart ausgebildet, dass vom Gewebe reflektiertes Licht in den optischen Leiter fokussiert wird.

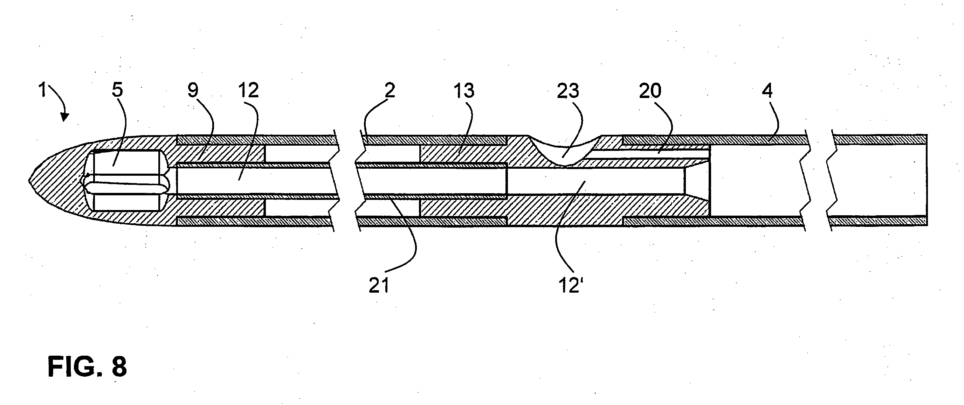

In Figur 8 ist ein Längsschnitt durch eine Messvorrichtung mit einem Mittelstück gemäss Figur 7 gezeigt, bei dem jedoch der Drainagekanal 12' zentriert angeordnet ist. Die Aussparung 16' reicht beinahe bis zum Drainagekanal 12', lässt diesen jedoch geschlossen. Die Wölbung oder Rundung der Aussparung 16' ist derart, dass Licht aus dem Kanal 20 direkt auf den Brennpunkt gerichtet wird. Die übrigen Elemente der Messvorrichtung dieser Ausführungsform entsprechen denjenigen aus den Figuren 6a und 6b.

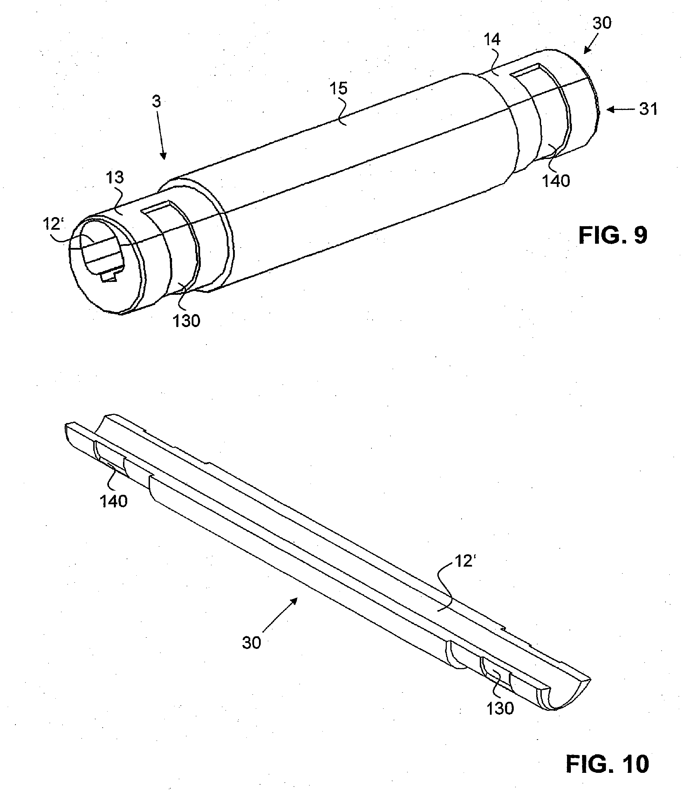

In den Figuren 9 bis 14 ist das erfindungsgemässe Mittelteil 3 in einer bevorzugten Ausführungsform dargestellt. Gleiche Teile sind mit gleichen Bezugszeichen wie in den obigen Beispielen versehen. Das Mittelteil 3 besteht aus Kunststoff und ist vorzugsweise im Spritzgussverfahren gefertigt. Es besteht aus mindestens zwei Teilen, einem Unterteil 31 und einem Deckel 30. Beide sind als Halbröhre ausgebildet, wie dies in Figur 10 für den Deckel 30 erkennbar ist. Gemeinsam ergeben sie eine Röhre mit einem Mittelbereich 15, einem distalen Befestigungsstück 13 und einem proximalen Befestigungsstück 14. An beiden Befestigungsstücken 13, 14 sind radial über dem Umfang verlaufende Nuten 130, 140 vorhanden, um eine Verbindung mit angrenzenden Schläuchen zu ermöglichen. Diese Nuten dienen als Klebenuten für einen Form- und Kraftschluss. Eine gleichartige Nut ist in Figur 17 erkennbar.

Anstelle von flexiblen und biegeweichen Schläuchen können auch auf beiden oder auf einer Seite starre Anschlussteile vorhanden sein.

Im Gegensatz zu den obigen Beispielen wird nun die Ausnehmung, in welcher der Spiegel, das Faserende und allfällige Messmittel angeordnet sind, vom Deckel 30 überdeckt. Der Deckel 30 kann form- und/oder kraft- und/oder stoffschlüssig mit dem Unterteil verbunden sein.

Vorzugsweise sind Deckel 30 und Unterteil 31 aus demselben Material gefertigt. Zumindest der Deckel 30 muss jedoch aus einem für die verwendete optische Wellenlänge transparenten oder durchlässigem Material gefertigt sein. Vorzugsweise bestehen sie aus Polyamid oder Polycarbonat.

Wie in Figur 9 erkennbar ist, bilden das Unterteil 31 und der Deckel 30 einen gemeinsamen inneren Drainagekanal 12' mit einem Kabelkanal 120. Durch diesen Drainagekanal 12' verläuft die bereits oben beschriebene Drainageleitung und allfällige Messleitungen bis zum distalen Kopfteil des Katheters.

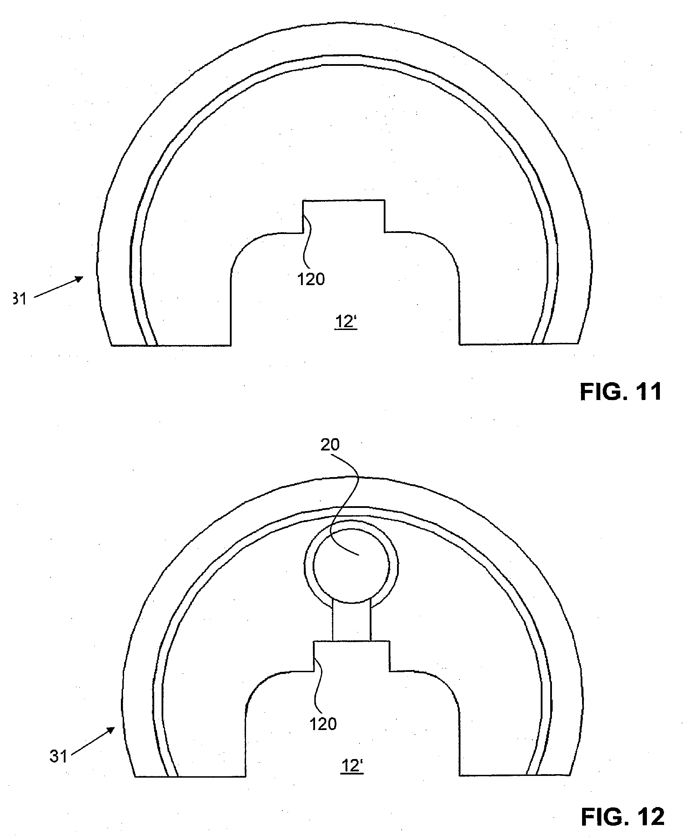

Figur 11 zeigt das distale Ende des Mittelteils 3 mit dem Drainagekanal 12'. In Figur 12 ist das in Figur 9 nicht sichtbare innere proximale Ende des Mittelteils 3 sichtbar. Ein Lichtleiterkanal 20 verläuft parallel zum Drainagekanal 12' aber beabstandet zu diesem. Auch hier wird vorzugsweise genau eine optische Faser als Lichtleiter verwendet. Es können aber auch bis zu fünf, insbesondere zwei oder drei optische Fasern sein.

Unterhalb der Aussparung 16 verläuft über die gesamte Länge des Mittelstücks 3 der Drainagekanal 12'.

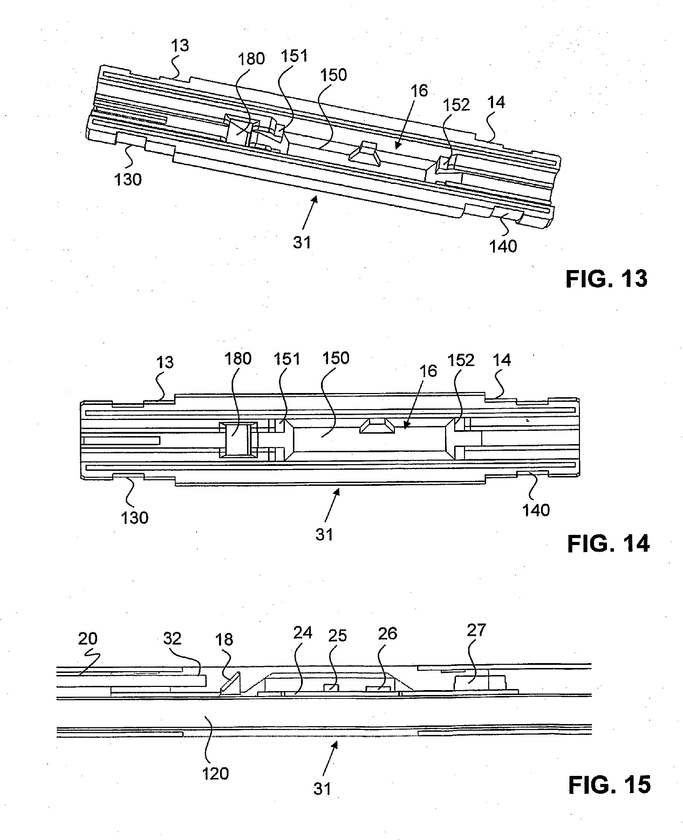

In den Figuren 13 bis 15 ist das Unterteil 31 im Detail dargestellt. Es weist die Aussparung 16 auf. In dieser Aussparung 16 ist ein Aufnahmebereich 150 für die Elektronik vorhanden. Proximale und distale Aufnahmeflächen 151, 152 sind an beiden Endbereichen des Aufnahmebereichs 150 angeformt. Auf diese planen Aufnahmeflächen 151, 152 lässt sich eine Leiterplatine 24 befestigen, beispielsweise ankleben oder verschrauben.

Auf der Leiterplatine 24 lässt sich ein Photodetektor 25 befestigen. Wahlweise kann zudem ein Temperatursensor 26 und/oder ein Drucksensor 27 vorhanden sein. Diese Sensoren können jedoch auch an einer anderen Stelle angeordnet sein. Wie bereits oben anhand der anderen Beispiele erwähnt, ist der Drucksensor vorzugsweise im Spitzenelement 1 angeordnet.

Ausserhalb dieses Aufnahmebereichs 150 ist eine Schrägfläche 180 vorhanden. Diese Schrägfläche 180 kann selber als Reflexionsfläche ausgebildet sein. Vorzugsweise dient sie jedoch als Halterung für einen Spiegel 18. Der Spiegel 18 ist in diesem Beispiel ein planparalleler Spiegel, so dass auch die Schrägfläche 180 als plane Fläche ausgebildet ist. Vorzugsweise ist die Schrägfläche 180 unter einem 45° Winkel zur Längsrichtung des Mittelstücks 3 ausgerichtet.

Der Lichtleiterkanal 20 verläuft im Unterteil 31 und endet benachbart zur Schrägfläche 180. In Figur 15 ist der Lichtleiter 32 dargestellt. Es ist erkennbar, dass er vor dem Spiegel 18 endet, so dass vom Lichtleiter 32 ausgestrahltes Licht vom Spiegel 18 umgelenkt und radial nach aussen gesandt wird.

Im Gewebe reflektiertes Licht wird in diesem Beispiel nicht mehr über den Lichtleiter zurückgeführt und ausgewertet. Es wird vielmehr vom Photodetektor detektiert und ein elektrisches Signal wird über eine Signalleitung an eine externe Auswerteinheit übermittelt. Die hierzu notwendigen Kabel verlaufen im Kabelkanal 120.

Dieses Mittelteil 3 lässt sich mit einem der oben beschriebenen Spitzenelemente 1 verwenden. Es lässt sich jedoch auch mit anderen Spitzenelementen 1 einsetzen.

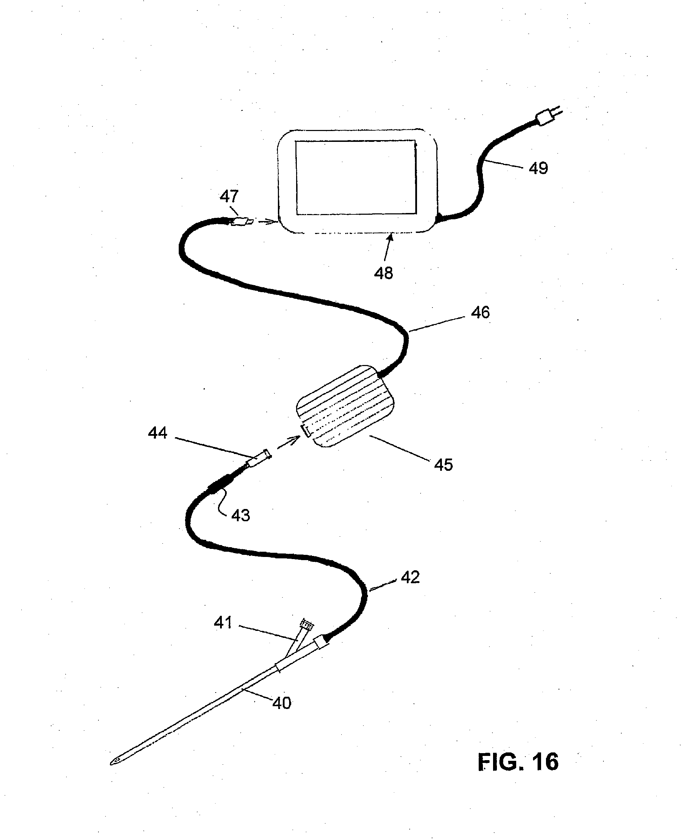

Figur 16 zeigt das Gesamtsystem. Der Katheter oder die Sonde 40 ist am Ende des Drainagekanals mit einem Luer-Lock 41 versehen. Eine erste Leitung 42, welche sowohl elektrische wie mindestens einen optischen Leiter umfasst, ist mit einem distalen Ende mit der Sonde 40 verbunden. Im Bereich des proximalen Endes weist sie eine Kalibrierungseinheit 43 auf und sie ist an diesem Ende mit einem ersten Stecker 44 versehen. Dieser Stecker 44 ermöglicht eine Verbindung zu eine portablen Elektronikeinheit 45. Diese Elektronikeinheit 45 umfasst vorzugsweise eine Potentialtrennung, einen A/D Wandler, einen Vorverstärker und eine Lichtquelle zur Einkopplung von Licht in den optischen Leiter, welcher zum Mittelteil 3 des Katheters führt.

Die portable Elektronikeinheit 45 ist über eine zweite rein elektrische Leitung 46, welche wiederum mit einem zweiten Stecker 47 versehen ist, zu einer Auswerteinheit 48. Eine dritte Leitung 49 stellt das Stromnetzkabel dar. Die Auswerteinheit 48 kann jedoch selbstverständlich auch batteriebetrieben sein.

Der Vorteil dieses Gesamtsystems besteht darin, dass die Elektronikeinheit 45 relativ nahe beim Patienten ist. Die empfindlichen optischen Leitungen können somit relativ kurz ausgebildet sein.

In den oben beschriebenen Beispielen wird vorzugsweise eine optische Glasfaser im nahen Infrarotbereich verwendet. Ihr Durchmesser beträgt vorzugsweise annähernd 0.6 mm inkl. ihrer Umhüllung und annähernd 0.2 mm ohne Umhüllung. Die Länge des Mittelstücks 3 beträgt vorzugsweise 20 bis 30 mm und sein Durchmesser beträgt vorzugsweise 3 mm. Die Spiegelfläche beträgt vorzugsweise 1x1 mm. Der Drainagekanal weist vorzugsweise einen Durchmesser von 0.9 bis 1.5 mm, vorzugsweise 1.0 oder 1.2 mm auf. Die Breite der Platine beträgt vorzugsweise 1.4 mm. Innerhalb des Mittelstücks 3 wird üblicherweise ein 9 oder 10-poliges Kabel verwendet, welches sich bis zum Stecker 44 erstreckt.

### Bezugszeichenliste

- 1: Spitzenelement
- 2: Erster Schlauchteil
- 3: Mittelstück
- 30: Deckel
- 31: Unterteil
- 32: Lichtleiter
- 4: Zweiter Schlauchteil
- 5: längliche Öffnung
- 6: runde Öffnung
- 8: Spitze
- 9: Befestigungsstück
- 10, 10': Drucksensorkanal
- 11: Steg
- 12, 12': Drainagekanal
- 120: Kabelkanal
- 13: Befestigungsstück distal
- 130: distale Klebenut
- 14: Befestigungsstück proximal
- 140: proximale Klebenut
- 15: Mittelbereich
- 150: Aufnahme für Elektronik
- 151: proximale Aufnahmefläche
- 152: distale Aufnahmefläche
- 16, 16': Ausnehmung, Einschnitt, Aussparung
- 17: Lichtaustrittsfläche
- 18: Reflexionsfläche
- 180: Schrägfläche
- 20: Lichtleiterkanal
- 21: Schlauch innen
- 22: Temperaturmesskanal
- 23: Innenfläche mit Lichtaustrittsflächen- und Reflexionsflächenbereich
- 24: Leiterplatine
- 25: Photodetektor
- 26: Temperatursensor
- 27: Drucksensor
- 40: Sonde
- 41: Luer-Lock
- 42: erste Leitung (optoelektronisch)
- 43: Kalibrierung
- 44: erster Stecker
- 45: portable Elektronikeinheit
- 46: zweite Leitung (rein elektrisch)
- 47: zweiter Stecker
- 48: Auswerteeinheit
- 49: dritte Leitung

## Patentansprüche

1. Katheter zur Messung einer Durchblutung in einem Körpergewebe, wobei im Katheter ein Lichtemitter (32) zur Emission von Licht in das Körpergewebe und ein Lichtempfänger (32, 25) zum Empfang von im Körpergewebe reflektierten und zum Katheter zurückgestrahlten Licht angeordnet sind, **dadurch gekennzeichnet, dass** der Katheter ein Mittelstück (3) und ein erstes und ein zweites Verbindungsrohr (2, 4) umfasst, wobei das Mittelstück (3) ein distales Befestigungsstück (13) zur Befestigung am ersten Verbindungsrohr (2) und ein proximales Befestigungsstück (14) zur Befestigung am zweiten Verbindungsrohr (4) aufweist, und dass der Lichtemitter (32) und der Lichtempfänger (32, 25) im Mittelstück (3) angeordnet sind.

2. Katheter nach Anspruch 1, wobei das Mittelstück (3) starr ausgebildet ist.

3. Katheter nach einem der Ansprüche 1 oder 2, wobei der Katheter ferner ein starres Kopfteil (1) umfasst, wobei das Kopfteil (1) ein drittes Befestigungsstück (9) aufweist zur Befestigung am ersten Verbindungsrohr (2), so dass das Kopfteil (1) beabstandet zum Mittelstück (3) angeordnet ist.

4. Katheter nach einem der Ansprüche 1 bis 3, wobei mindestens das erste Verbindungsrohr (2) flexibel ausgebildet ist.

5. Katheter nach einem der Ansprüche 1 bis 4, wobei das Mittelstück (3) aus Metall oder Kunststoff gefertigt ist.

6. Katheter nach einem der Ansprüche 1 bis 5, wobei das Mittelstück (3) eine Ausnehmung (16, 16') aufweist, in welcher der Lichtemitter (32) und der Lichtempfänger (32, 25) angeordnet sind.

7. Katheter nach Anspruch 6, wobei das Mittelstück (3) einen Deckel (30) aufweist, welcher die Ausnehmung (16, 16') überdeckt.

8. Katheter nach einem der Ansprüche 1 bis 7, wobei der Lichtemitter ein optischer Leiter (32) ist, welcher ein distales Ende aufweist und wobei das distale Ende im Mittelstück (3) gehalten ist.

9. Katheter nach einem der Ansprüche 1 bis 8, wobei im Mittelstück (3) ein Drucksensor (27) und/oder ein Temperatursensor (26) angeordnet sind.

10. Katheter nach einem der Ansprüche 1 bis 9, wobei der Lichtempfänger ein Photodetektor (25) ist, welcher im Mittelstück (3) angeordnet ist.

11. Katheter nach den Ansprüchen 6 und 10, wobei in der Ausnehmung (16, 16') eine Aufnahme (150) vorhanden ist, in welcher eine Leiterplatine (24) befestigt ist und wobei mindestens der Photodetektor (25) auf der Leiterplatte (24) angeordnet ist.

12. Katheter nach einem der Ansprüche 1 bis 11, wobei im Mittelstück (3) ein Lichtreflexionsflächenbereich, insbesondere ein Spiegel (18), vorhanden ist, welcher einem Lichtaustrittflächenbereich (17) des Lichtemitters (32) gegenüberliegt und welcher aus dem Lichtemitter (32) austretendes Licht in das Körpergewebe umlenkt.

13. Katheter nach Anspruch 12, wobei das Mittelstück (3) eine Schrägfläche (180) aufweist, auf welcher ein Spiegel (18) befestigt ist, wobei der Spiegel (18) den Lichtreflexionsflächenbereich bildet.

14. Katheter nach einem der Ansprüche 1 bis 13, wobei er einen Drainagekanal (12, 12') aufweist, welcher durch das Mittelstück (3) verläuft.

15. Katheter gemäss einem der Ansprüche 1 bis 14, wobei der Katheter einen Katheterkopf (1) aufweist zum Einführen in ein Körpergewebe, der sich in einen Einführbereich und einen sich daran anschliessenden Anschlussbereich unterteilt, wobei der Einführbereich in seiner Oberfläche mehrere Aussparungen (5) umfasst, wobei
- der Einführbereich in Richtung des Anschlussbereichs einen zunehmenden Durchmesser aufweist und
- die Aussparungen (5) derart im Einführbereich vorgesehen sind, dass zwischen den Aussparungen entlang der Oberfläche des Katheterkopfes Stege (11) in Richtung des Anschlussbereichs verlaufend ausgebildet sind, die in einem Bereich des Einführbereichs mit geringem Durchmesser beginnen und in einem Bereich mit grösserem Durchmesser enden.

16. Katheter nach Anspruch 15, **dadurch gekennzeichnet, dass** der Einführbereich eine parabelförmige Spitze (8) aufweist.

17. Vorrichtung zur Messung der Durchblutung in einem Körpergewebe, umfassend:
- einen Katheter gemäss einem der Ansprüche 15 bis 16,
- einen optischen Leiter (32) innerhalb des Katheters, gemäß Anspruch 8,
- eine Lichtquelle zur Emission eines Lichtstrahls in das Körpergewebe mittels des optischen Leiters,
- eine Verarbeitungseinheit (48) zur Ermittlung der Durchblutungsrate mittels eines aus dem Körpergewebe reflektierten Lichtstrahls,
**dadurch gekennzeichnet, dass**
- der Katheter ein Mittelstück (3) mit einer Ausnehmung (16; 16') gemäß Anspruch 6 aufweist, die einen Lichtaustrittsflächenbereich (17), aus dem der optische Leiter mündet, und einen Reflexionsflächenbereich (18) gemäß Anspruch 12 umfasst, der dem Lichtaustrittsflächenbereich (17) gegenüberliegt und der zumindest teilweise schräg zur Längsachse des optischen Leiters ausgerichtet ist,
- wobei der optische Leiter derart angeordnet ist, dass ein aus der Lichtquelle emittierter Lichtstrahl auf den Reflexionsflächenbereich (18) gerichtet ist, der emittierte Lichtstrahl an dem Reflexionsflächenbereich (18) umlenkbar und in das umliegende Körpergewebe reflektierbar ist und ein an dem Körpergewebe reflektierter Lichtstrahl an dem Reflexionsflächenbereich (18) reflektierbar ist und in den einen optischen Leiter eingespeist wird.

## Claims

1. Catheter for measuring a flow of blood through a body tissue, wherein a light emitter (32) for emitting light into the body tissue and a light receiver (32, 25) for receiving light reflected in the body tissue and back to the catheter are arranged in the catheter, **characterized in that** the catheter comprises a middle piece (3) and a first and a second connection tube (2, 4), wherein the middle piece (3) has a distal fastening piece (13) for fastening to the first connection tube (2) and a proximal fastening piece (14) for fastening to the second connection tube (4), and **in that** the light emitter (32) and the light receiver (32, 25) are arranged in the middle piece (3).

2. Catheter according to Claim 1, wherein the middle piece (3) is rigid.

3. Catheter according to either of Claims 1 and 2, wherein the catheter further comprises a rigid head part (1), wherein the head part (1) has a third fastening piece (9) for fastening to the first connection tube (2), such that the head part (1) is arranged at a distance from the middle piece (3).

4. Catheter according to one of Claims 1 to 3, wherein at least the first connection tube (2) is flexible.

5. Catheter according to one of Claims 1 to 4, wherein the middle piece (3) is made of metal or plastic.

6. Catheter according to one of Claims 1 to 5, wherein the middle piece (3) has a recess (16, 16') in which the light emitter (32) and the light receiver (32, 25) are arranged.

7. Catheter according to Claim 6, wherein the middle piece (3) has a lid (30) that covers the recess (16, 16').

8. Catheter according to one of Claims 1 to 7, wherein the light emitter is an optical conductor (32) that has a distal end, and wherein the distal end is held in the middle piece (3).

9. Catheter according to one of Claims 1 to 8, wherein a pressure sensor (27) and/or a temperature sensor (26) are arranged in the middle piece (3).

10. Catheter according to one of Claims 1 to 9, wherein the light receiver is a photodetector (25) arranged in the middle piece (3).

11. Catheter according to Claims 6 and 10, wherein a receptacle (150) in which a circuit board (24) is secured is present in the recess (16, 16'), and wherein at least the photodetector (25) is arranged on the circuit board (24).

12. Catheter according to one of Claims 1 to 11, wherein a light reflection surface area, in particular a mirror (18), is present in the middle piece (3), lies opposite a light exit surface area (17) of the light emitter (32) and deflects light issuing from the light emitter (32) into the body tissue.

13. Catheter according to Claim 12, wherein the middle piece (3) has an inclined surface (180) on which a mirror (18) is secured, wherein the mirror (18) forms the light reflection surface area.

14. Catheter according to one of Claims 1 to 13, wherein it has a drainage channel (12, 12') extending through the middle piece (3).

15. Catheter according to one of claims 1 to 14, wherein the catheter comprises a catheter head (1) for insertion into a body tissue, divided into an insertion area and an adjoining connection area, the insertion area comprising several apertures (5) in its surface, wherein
- the insertion area has a diameter that increases in the direction of the connection area, and
- the apertures (5) are provided in the insertion area in such a way that webs (11) extending in the direction of the connection area are formed between the apertures along the surface of the catheter head, which webs (11) begin in an area of the insertion area with a small diameter and end in an area with a greater diameter.

16. Catheter head according to claim 15, wherein the insertion area has a parabola-shaped tip (8).

17. Device for measuring the flow of blood through a body tissue, comprising:
- a catheter according to one of claims 15 to 16,
- an optical conductor (32) inside the catheter, according to claim 8,
- a light source for emitting a beam of light into the body tissue by means of the optical conductor,
- a processing unit (48) for determining the rate of blood flow by means of a beam of light reflected from the body tissue,
**characterized in that**
- the catheter has a middle piece (3) with a recess (16, 16') according to claim 6, comprising a light exit surface area (17), from which the optical conductor emerges, and a reflection surface area (18) according to claim 12 which lies opposite the light exit surface area (17) and which is oriented at least partially obliquely with respect to the longitudinal axis of the optical conductor,
- the optical conductor being arranged in such a way that a beam of light emitted from the light source is directed to the reflection surface area (18), the emitted beam of light can be deflected on the reflection surface area (18) and can be reflected into the surrounding body tissue, and a beam of light reflected on the body tissue can be reflected on the reflection surface area (18) and fed into the optical conductor.

## Revendications

1. Cathéter destiné à mesurer l'irrigation sanguine d'un tissu corporel,
un émetteur de lumière (32) destiné à émettre de la lumière dans le tissu corporel et un récepteur de lumière (32, 25) destiné à recevoir la lumière réfléchie dans le tissu corporel et renvoyée vers le cathéter étant disposés dans le cathéter,
**caractérisé en ce que**
le cathéter comporte une pièce centrale (3) ainsi qu'un premier et un deuxième tube de liaison (2, 4),
**en ce que** la pièce centrale (3) présente une pièce distale de fixation (13) destinée à être fixée sur le premier tube de liaison (2) et une pièce proximale de fixation (14) destinée à être fixée sur le deuxième tube de liaison (4) et
**en ce que** l'émetteur de lumière (32) et le récepteur de lumière (32, 25) sont disposés dans la pièce centrale (3).

2. Cathéter selon la revendication 1, dans lequel la pièce centrale (3) est rigide.

3. Cathéter selon l'une des revendications 1 ou 2, le cathéter présentant en outre une partie rigide de tête (1), la partie de tête (1) présentant une troisième pièce de fixation (9) destinée à être fixée sur le premier tube de liaison (2) de telle sorte que la partie de tête (1) soit disposée à distance de la pièce centrale (3).

4. Cathéter selon l'une des revendications 1 à 3, dans lequel au moins le premier tube de liaison (2) est flexible.

5. Cathéter selon l'une des revendications 1 à 4, dans lequel la pièce centrale (3) est réalisée en métal ou en matière synthétique.

6. Cathéter selon l'une des revendications 1 à 5, dans lequel la pièce centrale (3) présente une découpe (16, 16') dans laquelle l'émetteur de lumière (32) et le récepteur de lumière (32, 25) sont disposés.

7. Cathéter selon la revendication 6, dans lequel la pièce centrale (3) présente un couvercle (30) qui recouvre la découpe (16, 16').

8. Cathéter selon l'une des revendications 1 à 7, dans lequel l'émetteur de lumière est un conducteur optique (32) qui présente une extrémité distale, l'extrémité distale étant maintenue dans la pièce centrale (3).

9. Cathéter selon l'une des revendications 1 à 8, dans lequel une sonde de pression (27) et/ou une sonde de température (26) sont disposées dans la pièce centrale (3).

10. Cathéter selon l'une des revendications 1 à 9, dans lequel le récepteur de lumière est un photodétecteur (25) disposé dans la pièce centrale (3).

11. Cathéter selon les revendications 6 et 10, dans lequel un logement (150) dans lequel une carte de circuit (24) est fixée est prévu dans la découpe (16, 16'), le photodétecteur (25) étant disposé sur la carte de circuit (24).

12. Cathéter selon l'une des revendications 1 à 11, dans lequel une zone de surface de réflexion de lumière, en particulier un miroir (18) est prévu dans la pièce centrale (3), est situé face à une zone (17) de surface de sortie de lumière de l'émetteur de lumière (32) et dévie dans le tissu corporel la lumière sortant de l'émetteur de lumière (32).

13. Cathéter selon la revendication 12, dans lequel la pièce centrale (3) présente une surface oblique (180) sur laquelle un miroir (18) est fixé, le miroir (18) formant la zone de surface de réflexion de lumière.

14. Cathéter selon l'une des revendications 1 à 13, qui présente un canal de drainage (12, 12') qui s'étend à travers la pièce centrale (3).

15. Cathéter selon l'une des revendications 1 à 14, dans lequel le cathéter présente une tête (1) de cathéter destinée à l'insertion dans un tissu corporel et se divisant en une partie d'insertion et une partie de raccordement qui lui est adjacente, la partie d'insertion présentant dans sa surface plusieurs découpes (5),
la partie d'insertion présentant un diamètre croissant en direction de la partie de raccordement et
les découpes (5) étant prévues dans la partie d'insertion de telle sorte que des nervures (11) qui commencent au niveau de la partie d'insertion de petit diamètre et se terminent dans une zone de plus grand diamètre soient formées entre les découpes, sur la surface de la tête du cathéter, s'étendent en direction de la partie de raccordement.

16. Cathéter selon la revendication 15, **caractérisé en ce que** la zone d'insertion présente une pointe (8) de forme parabolique.

17. Dispositif de mesure de l'irrigation sanguine d'un tissu corporel, le dispositif présentant :
un cathéter selon l'une des revendications 15 à 16,
un conducteur optique (32) situé à l'intérieur du cathéter selon la revendication 8,
une source de lumière qui émet un faisceau lumineux dans le tissu corporel au moyen du conducteur optique,
une unité de traitement (48) qui détermine le débit d'irrigation sanguine au moyen d'un faisceau lumineux réfléchi par le tissu corporel,
**caractérisé en ce que**
le cathéter présente une pièce centrale (3) dotée d'une découpe (16, 16') selon la revendication 6, qui comporte une zone (17) de surface de sortie de lumière de laquelle débouche le conducteur optique et une zone (18) de surface de réflexion selon la revendication 12, située face à la zone (17) de surface de sortie de lumière et orientée au moins en partie obliquement par rapport à l'axe longitudinal du conducteur optique,
**en ce que** le conducteur optique est disposé de telle sorte qu'un faisceau lumineux émis par la source de lumière soit dirigé sur la zone (18) de surface de réflexion, que le faisceau lumineux émis puisse être dévié sur la zone (18) de surface de réflexion pour être réfléchi dans le tissu corporel environnant et qu'un faisceau lumineux réfléchi par le tissu corporel puisse être réfléchi sur la zone (18) de surface de réflexion et soit injecté dans l'un des conducteurs optiques.
